# FASCICULE DE BREVET EUROPEEN

(11) **EP 2 285 274 B1**
(45) Date de publication et mention de la délivrance du brevet: **11.07.2012**
(21) Numéro de dépôt: 09750003.7
(22) Date de dépôt: 05.05.2009
(51) Int. Cl.: A61B 5/103, A61H 19/00, A61N 1/05, A61F 5/41, A61B 5/024, A61N 1/36, A63B 23/00

(54) **DISPOSITIF ET PROCEDE DE MESURE DE LA RIGIDITE ET DE L'ERECTION D'UN PENIS ET DES DEBITS ARTERIO-VEINEUX**
VORRICHTUNG UND VERFAHREN ZUR MESSUNG DER STEIFHEIT UND EREKTION EINES PENIS SOWIE DER ARTERIOVENÖSEN ZIRKULATION
DEVICE AND METHOD FOR MEASURING THE STIFFNESS AND ERECTION OF A PENIS AND THE ARTERIOVENOUS CIRCULATION

(30) Priorité: 05.05.2008 FR 0802473
(43) Date de publication de la demande: 23.02.2011
(73) Titulaire: Lavoisier, Pierre, 84110 Rasteau (FR)
(72) Inventeur: Lavoisier, Pierre, 84110 Rasteau (FR)
(74) Mandataire: Maureau, Philippe
(86) Numéro de dépôt international: PCT/FR2009/000521
(87) Numéro de publication internationale: WO 2009/141522

(56) Documents cités:
- EP-A- 1 350 465
- WO-A-96/20753
- WO-A-98/38914
- DE-A1-102004 023 966
- JP-A- 2006 149 566
- US-A- 4 925 619

## Description

La présente invention est relative à un procédé de mesure de la rigidité, de l'érection d'un pénis et des débits artério-veineux, ainsi qu'un dispositif pour la mise en oeuvre de ce procédé.

On connait d'après le brevet européen EP 1 350 465 au nom du demandeur un dispositif de mesure de type brassard permettant de mesurer la rigidité d'un pénis.

On connait d'après le brevet allemand DE 10 2004 012966 un dispositif destiné à détecter une grandeur caractéristique dépendant d'une fonction physiologique comme par exemple de localiser un tissu nerveux spécifique et des nerfs lors d'une opération du périnée. Ce dispositif est constitué d'une unité porteuse de forme annulaire ouvert 20 composée d'un matériau élastique, et dont le diamètre interne est réglable à l'aide d'une fermeture agrippante 42. L'unité porteuse 20 est raccordée à une unité électronique 34 qui comporte des moyens de détection 10, une mémoire 28 et une puce. Ces moyens de détection 10 permettent de détecter des grandeurs caractéristiques dépendant d'une modification du diamètre du pénis 18 et non de la pression.

Le dispositif et le procédé de mesure suivant la présente invention ont pour objet de mesurer l'état de fatigue des muscles ischio-caverneux (IC), de mesurer les variations de la force musculaire des muscles ischio-caverneux (IC), et de mesurer le module de Young du corps caverneux (CC) afin de permettre d'évaluer la compliance dudit corps caverneux.

Ces mesures réalisées par le dispositif sont effectuées soit au cours du sommeil du patient soit le jour et permettent en fonction des résultats obtenus de déterminer la cause des dysfonctions érectiles et d'en contrôler l'évolution.

Également, le dispositif de mesure objet de l'invention permet de réaliser la surveillance de la rééducation des patients dans le cas d'éjaculation précoce et/ou de dysfonctions érectiles.

Il est connu de mesurer l'érection nocturne du pénis, afin de pouvoir dissocier les impuissances organiques des impuissances psychogènes. Les patients ayant une impuissance organique n'ont pas d'érection de jour, ni durant le sommeil paradoxal, tandis que les patients psychogènes ont des érections au cours du sommeil, mais n'en ont pas le jour.

Le procédé de mesure de la force des muscles ischio-caverneux (IC) provoquant la rigidité du pénis (P) d'un patient et de la variation du débit artériel et veineux assurant l'érection du pénis (P) du patient consiste à :
- Placer un capteur autour du pénis (P) comportant des moyens pour capter la pression et les variations de la pression intra caverneuse (PIC) du pénis (P), et au moins un transducteur intégré ou déporté du capteur permettant de transformer le signal de pression issu des moyens pour capter la pression en un signal électrique,
- Et à connecter le capteur à un boîtier comportant des moyens d'amplification et d'enregistrement du signal provenant du transducteur, des moyens d'analyse du signal par un microcontrôleur permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

Le procédé de mesure suivant la présente invention consiste à mesurer les variations de la pression intra caverneuse (PIC) du pénis (P) afin de quantifier la détumescence, la tumescence et la rigidité de jour comme de nuit du pénis (P).

Le procédé de mesure suivant la présente invention consiste à mesurer la pression intra caverneuse (PIC) qui atteint progressivement la pression systolique moyenne du pénis (P).

Le procédé de mesure suivant la présente invention consiste à mesurer, pendant la phase vasculaire qui est la première phase de l'érection du pénis (P), l'augmentation du débit artériel d'entrée dans le corps caverneux (CC) et la diminution du débit veineux de sortie.

Le procédé de mesure suivant la présente invention consiste à mesurer, pendant la phase musculaire qui est la seconde phase de l'érection du pénis (P), les variations de la contraction et de la rigidité des muscles ischio-caverneux (IC) entourant le corps caverneux (CC).

Le procédé de mesure suivant la présente invention consiste à mesurer, en continu, notamment au cours du sommeil du patient, le débit artériel d'entrée et le débit veineux de sortie par la mesure de la pression du pouls (PP) et du volume du pouls (VP).

Le procédé de mesure suivant la présente invention consiste à mesurer le module de Young du corps caverneux (CC) permettant d'évaluer la compliance dudit corps caverneux.

Le procédé de mesure suivant la présente invention consiste à mesurer la fuite caverno veineuse à partir de la courbe de la pression intra caverneuse (PIC).

Le procédé de mesure suivant la présente invention consiste à mesurer la contraction maximum (Pmax), le gradient de pression (Delta P et delta Moyen), la surface sous la courbe de chaque contraction correspondant au travail musculaire, le nombre de pics et la largeur de chaque pic.

Le procédé de mesures suivant la présente invention consiste à mesurer la fatigue des muscles ischio-caverneux (IC) en mesurant l'angle (α).

Le procédé de mesure suivant la présente invention consiste à mesurer les pics des contractions des muscles ischio-caverneux (IC) et les variations de pression du pouls en conservant une pression constante dans le capteur quelle que soit la variation du volume du pénis.

Le dispositif de mesure pour la mise en oeuvre du procédé de mesures suivant la présente invention comprend un capteur qui est constitué :
- D'un brassard solidaire sur sa face interne d'un réservoir rempli d'un liquide permettant de capter la pression et les variations de pression intra caverneuse (PIC),
- D'électrodes de stimulations positionnées entre le réservoir et la paroi externe du pénis (P) du patient,
- Et des tubulures de connexion raccordant le réservoir à un photopléthysmographe et à un transducteur de pression qui sont connectés au boîtier permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

Le dispositif de mesure suivant la présente invention comprend un brassard qui est connecté par l'intermédiaire d'une première vanne trois voies au photopléthysmographe mesurant les déplacements d'une membrane souple solidaire de la troisième tubulure de connexion afin de mesurer le volume du pouls pénien provenant des artères caverneuses.

Le dispositif de mesure suivant la présente invention comprend un brassard qui est connecté par l'intermédiaire de la seconde tubulure de connexion et d'une seconde vanne trois voies à un régulateur de pression pour assurer une pression constante à l'intérieur du réservoir du brassard.

Le dispositif de mesure suivant la présente invention comprend un régulateur de pression qui est constitué d'un cylindre comportant deux chambres remplies d'un fluide, séparées par une paroi percée d'un orifice calibré reliant lesdites chambres entre elles, ladite première chambre étant reliée à la vanne trois voies, tandis que ladite seconde chambre comporte un piston permettant de faire varier la pression du fluide au moyen d'un dispositif de vis micrométrique.

Le dispositif de mesure suivant la présente invention comprend une vis micrométrique qui comporte un ressort disposé autour de la tige de commande du piston afin de régler la tension dudit ressort et maintenir une pression stable dans le brassard tout en permettant des variations de pression élevées et de courte durée.

Le dispositif de mesure suivant la présente invention comprend un lien non élastique pour la retenue et la fermeture du brassard sécurisées autour du pénis du patient.

Le dispositif de mesure suivant la présente invention comprend un boîtier qui contient un amplificateur, un convertisseur A/D, un microprocesseur, et une carte mémoire amovible.

Le dispositif de mesure suivant la présente invention comprend un photopléthysmographe sensible à la pression qui est positionné à une certaine distance du réservoir pour éviter que les variations de pression dudit réservoir viennent perturber ledit photopléthysmographe.

Le dispositif de mesure suivant la présente invention comprend un photopléthysmographe qui comporte une diode photoémettrice et une cellule photoélectrique permettant d'envoyer un rayon lumineux en direction de la membrane souple et de recevoir le rayon réfléchi capté par la cellule, tandis que ledit signal reçu par le photopléthysmographie est transmis au boîtier qui permet l'amplification et l'enregistrement dudit signal.

Le dispositif de mesure pour la mise en oeuvre du procédé de mesures suivant la présente invention comprend un capteur qui est constitué :
- D'un anneau ouvert ou semi ouvert comportant deux branches en arc de cercle reliées entre elles par un élément souple, lesdites branches comportant sur leur face interne des zones de contacts constituées par des pastilles en caoutchouc présentant une faible déformation élastique et venant prendre appui contre le pénis (P) du patient de manière à décaler légèrement la face interne desdites branches de ce dernier.
- D'une jauge de contrainte solidaire de l'élément souple et connectée au boîtier permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

Le dispositif de mesure suivant la présente invention comprend des branches qui sont plates en forme de lamelle et réalisées dans un matériau ayant une grande résistance à la déformation élastique ou dit peu déformable.

Le dispositif de mesure suivant la présente invention comprend un élément souple reliant les branches qui est constitué d'une fine platine de métal présentant des caractéristiques de déformation élastique importante.

Le dispositif de mesure suivant la présente invention comprend un anneau ouvert qui comporte des moyens d'électro stimulation constitués d'électrodes disposées sur les pastilles en caoutchouc.

Les dessins annexés, donnés à titre d'exemple, permettront de mieux comprendre l'invention, les caractéristiques qu'elle présente et les avantages qu'elle est susceptible de procurer :
Figure 1 est une vue en perspective illustrant un dispositif de mesure comportant un capteur réalisé à partir d'un brassard non élastique suivant la présente invention.
Figure 2 est une vue schématique représentant un régulateur de pression qui peut être installé sur la tubulure d'alimentation du réservoir du dispositif de mesure et de surveillance suivant la présente invention.
Figures 3 à 5 sont des vues montrant un dispositif de mesure comportant un capteur réalisé à partir d'un anneau en position fermée dite « détumescence » suivant la présente invention.
Figure 6 est une vue illustrant l'anneau en position ouverte dite « d'érection » suivant la présente invention.
Figure 7 est un schéma montrant la variation de la pression mesurée par le dispositif de mesure suivant la présente invention.

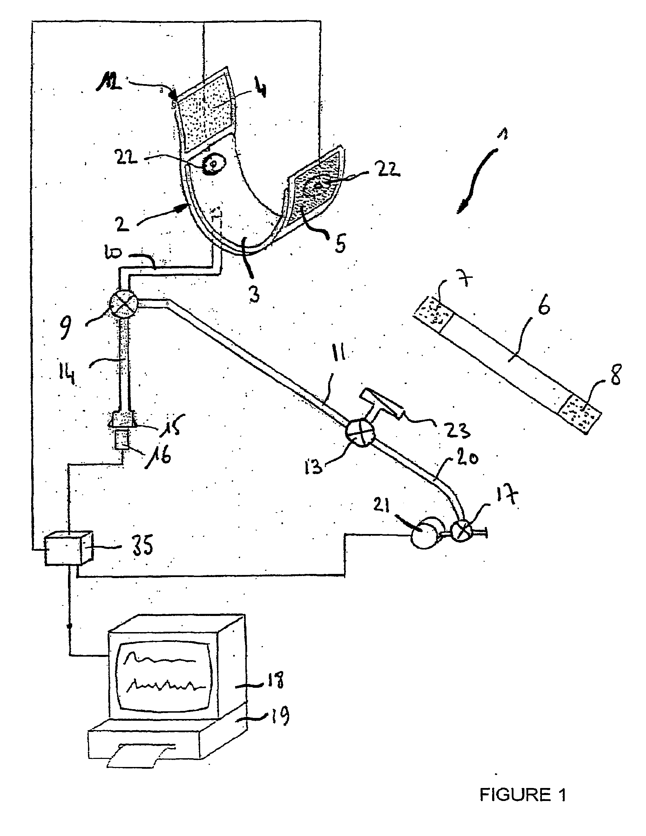

On a montré en figure 1 un dispositif de mesure 1 permettant de mesurer la pression intra-caverneuse et le débit sanguin au niveau du pénis P d'un patient.

Le dispositif de mesure 1 est constitué d'un capteur 2 formé d'un brassard 12 réalisé dans un matériau synthétique non élastique solidaire sur sa face interne d'un réservoir 3 en plastique rempli d'un fluide tel que de l'eau.

Le brassard 12 comporte à l'une des extrémités de sa face interne et dans le prolongement du réservoir 3 une première bande auto agrippante 4 coopérant avec une seconde bande auto agrippante 5 prévue à l'autre extrémité et sur la face externe dudit brassard 12.

Les premières et secondes bandes auto agrippantes 4, 5 coopèrent l'une avec l'autre pour assurer la fermeture sans déformation élastique du brassard 12 autour du pénis P.

La fermeture du brassard 12 autour du pénis est complétée par un lien non élastique 6 solidaire sur chacune de ses faces opposées et à chaque extrémité des bandes auto agrippantes 7, 8 permettant l'accrochage dudit lien 6 autour du brassard 12.

Le dispositif de mesure 1 comporte une première vanne trois voies 9 dont la première tubulure 10 est connectée de manière étanche à l'intérieur du réservoir 3 en matière plastique.

La première vanne trois voies 9 comporte une seconde tubulure 11 qui est raccordée à une seconde vanne trois voies 13 permettant la connexion à un régulateur de pression 23 et à une tubulure 20 reliée à une autre vanne trois voies 17 connecté à un capteur de pression ou transducteur 21.

La première vanne trois voies 9 comprend une troisième tubulure 14 qui est obturée par une membrane souple 15 réalisée par exemple en latex. Un photopléthysmographe 16 est placé à proximité de ladite membrane de manière à pouvoir mesurer les déplacements induits par le pouls artériel dans le corps caverneux du pénis P.

Le photopléthysmographe 16 comporte une diode photoémettrice et une cellule photoélectrique permettant d'envoyer un rayon lumineux en direction de la membrane souple 15 et de recevoir le rayon réfléchi capté par la cellule. Le signal reçu par le photopléthysmographe 16 est transmis à un boîtier 35 qui permet l'amplification et l'enregistrement dudit signal.

Le boîtier 35 contient un amplificateur, un convertisseur A/D, un microprocesseur, un système d'électrostimulation et une carte mémoire amovible. Le photopléthysmographe 16 étant sensible à la pression ce dernier est positionné à une certaine distance du réservoir 3, pour éviter que les variations de pression dudit réservoir viennent perturber ledit photopléthysmographe 16.

Egalement, la première vanne trois voies 9 permet, en fonction des mesures et des contrôles à réaliser, de connecter ou déconnecter le photopléthysmographe 16 permettant soit de l'utiliser soit de ne pas l'utiliser.

Le dispositif de mesure 1 comprend une troisième vanne trois voies 17 permettant la connexion du capteur de pression 21 pour mesurer la pression régnant dans le réservoir 3. Les signaux mesurés par le capteur de pression 21 sont transmis au boîtier 35 pour être amplifiés et enregistrés.

La troisième vanne trois voies 17 permet la mise en place d'une seringue, non illustrée, assurant le remplissage sans présence d'air dans les tubulures 10, 11, 14 et 20 et du réservoir 3 du dispositif de mesure 1.

Le dispositif de mesure 1 comporte des électrodes 22 qui sont positionnées sous le réservoir 3 de manière à être en contact avec la face externe du pénis P du patient lorsque le brassard 12 vient entourer ce dernier. Les électrodes 22 sont collées sur la peau du pénis P.

Les électrodes 22 sont connectées au boîtier 35 comprenant un système d'électro-stimulation permettant d'envoyer des impulsions électriques aux dites électrodes 22 afin d'assurer une électro-stimulation permettant une meilleur mesure des muscles ischio-caverneux IC entourant le corps caverneux du pénis P.

On note que la stimulation électrique des électrodes 22 est contrôlée par le patient de manière à être bien perçue sans jamais être douloureuse. Le patient peut à tout moment en faire varier l'intensité.

Le dispositif de mesure 1 comprend au niveau de la seconde vanne trois voies 13 un régulateur de pression 23 qui permet d'assurer une pression stable à l'intérieur du réservoir 3 du brassard 12.

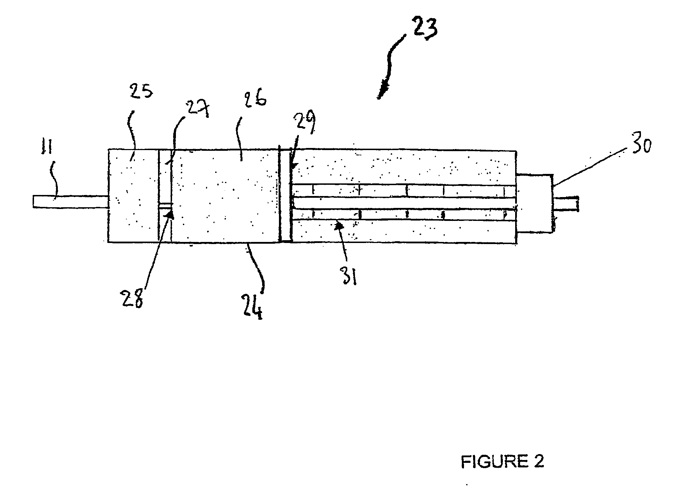

On a montré en figure 2 le régulateur de pression 23 qui est constitué d'un cylindre 24 comportant deux chambres 25, 26 remplies d'un fluide tel que de l'eau et séparées par une paroi 27 percée d'un orifice calibré 28 de faible diamètre reliant lesdites chambres entre elles.

La première chambre 25 est reliée par l'intermédiaire de la seconde vanne trois voies 13 aux tubulures du dispositif de mesure 1, tandis que la seconde chambre 26 comporte un piston 29 permettant de maintenir une pression stable à l'intérieur de ladite chambre 26, laquelle pression peut être réglée au moyen d'une vis micrométrique 30.

En effet, la vis micrométrique 30 agit sur un ressort 31 disposé autour de la tige de commande du piston 29 afin de régler la tension dudit ressort et donc la pression exercée par le piston 29 à l'intérieur de la seconde chambre 26.

Ainsi, les déplacements contrôlés du piston 29 à l'intérieur de la seconde chambre 26 permettent soit d'aspirer du fluide contenu dans la chambre 25, soit de rajouter du fluide à l'intérieur de cette dernière par l'intermédiaire de l'orifice calibré 28 et donc de maintenir une pression stable à l'intérieur du réservoir unique 3 et ceci quelles que soient les variations de volume du pénis P.

L'orifice calibré 28 permet au dispositif de mesure 1 d'enregistrer les variations de pression de courte durée au niveau du réservoir 3 et donc des pics de pression provenant des contractions des muscles ischio-caverneux IC du pénis P et les variations du pouls tout en conservant une pression stable dans le brassard 12 quelle que soit la variation de volume du pénis P évitant un serrage douloureux.

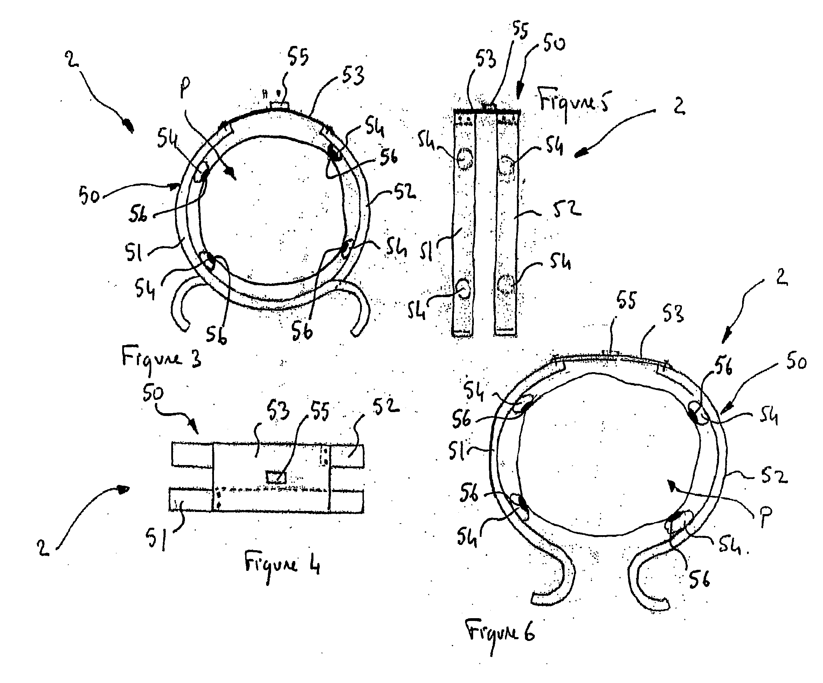

On a représenté en figures 3 à 6 un dispositif de mesure 1 comprenant un capteur qui est constitué d'un anneau ouvert ou semi ouvert 50 comportant deux branches 51, 52 en arc de cercle reliées entre elles au sommet du profil circulaire par un élément souple 53.

Les branches 51, 52 sont plates en forme de lamelle et réalisées dans un matériau ayant une grande résistance à la déformation élastique ou dite peu déformable.

L'anneau 50 comporte sur la face interne des branches 51, 52 des zones de contacts venant en appui contre le pénis de manière à décaler légèrement la face interne desdites branches de ce dernier. Les zones de contact sont réalisées par l'intermédiaire de pastilles en caoutchouc 54 présentant une faible déformation élastique afin de favoriser la circulation cutanée et de ne pas faire garrot au cours de l'érection.

L'anneau 50 comporte des moyens d'électro stimulation qui sont constitués d'électrodes 56 disposées sur les pastilles en caoutchouc 54 afin d'assurer une électro-stimulation permettant une meilleure mesure des muscles ischio-caverneux IC entourant le corps caverneux du pénis P.

L'élément souple 53 reliant les branches 51, 52 est constitué d'une fine platine de métal présentant des caractéristiques de déformation élastique importante. L'élément souple 53 comporte sur l'une de ses faces une jauge de contrainte 55 qui est connectée par tout moyen au boîtier 35 du dispositif de mesure 1.

Les branches 51, 52 de l'anneau 50 peuvent se croiser l'une par rapport à l'autre pour constituer un anneau fermé. Dans ce cas, il suffit d'agir sur l'extrémité libre des branches 51, 52 pour obtenir une légère déformation de l'élément souple 53 afin de pouvoir placer l'anneau 50 autour du pénis P du patient.

L'anneau 50 permet, du fait de sa structure et de sa composition, une adaptation aux différents volumes et circonférences de pénis P sans en gêner la circulation sanguine à l'intérieur de ce dernier.

Ainsi, les variations de volume du pénis P sont obtenues par l'ouverture plus ou moins grande de l'anneau 50 qui est mesurée par la déformation de la plaque souple ou platine 53 et enregistrée par la jauge 55.

Egalement, les variations de pressions sont obtenues par la déformation de la platine 53 en fonction de son élasticité et mesurées et enregistrées par la jauge 55.

L'anneau 50 permet du fait de sa conformation de passer d'une position fermée dite « détumescence » à une position ouverte dite « d'érection ». Les différentes positions de l'anneau 50 permettent de mesurer la variation de pression et de volume du pénis P, la compliance de ce dernier est donc delta V / delta P.

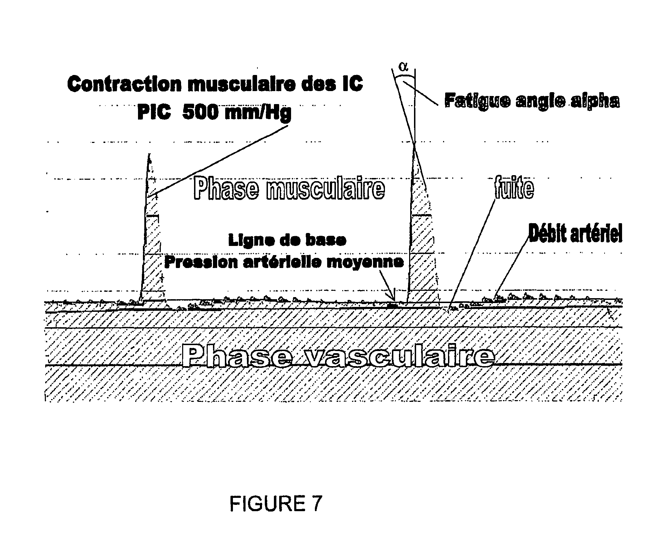

### ERECTION ET MESURES PHYSIOLOGIQUES (figure 7)

La qualité de l'érection pénienne n'est que le reflet de la pression intra caverneuse (PIC). La détumescence ou état de repos est la conséquence d'une faible pression intra caverneuse (PIC) de l'ordre de 10 à 20 mm/hg.

L'augmentation de la pression intra caverneuse (PIC) entraîne une augmentation du volume du pénis et de sa dureté.

La tumescence est la conséquence d'une pression intra caverneuse (PIC) moyenne de l'ordre de 100 mm/hg à 120 mm/hg.

La rigidité est la conséquence d'une pression intra caverneuse (PIC) élevée supérieure à 400mm/g.

L'augmentation de volume lors d'une élévation de la pression intra caverneuse (PIC) est proportionnelle à l'élasticité du corps caverneux (CC) définissant la compliance delta V / delta P.

Au cours d'une érection, la pression intra caverneuse (PIC) atteint progressivement la pression systolique moyenne soit 100 à 120 mm/hg. Le volume maximum du pénis est atteint lorsque l'élasticité pénienne est bloquée par l'albuginée.

L'albuginée est une membrane de faible compliance qui entoure le corps caverneux (CC) empêchant sa dilatation, ce qui permet l'élévation de pression.

La phase vasculaire est la première phase de l'érection, c'est la tumescence, conséquence de l'augmentation des débits artério-veineux (DAV) à savoir du débit d'entrée (artériel) dans le corps caverneux (CC) associée à une diminution du débit de sortie (veineux).

La seconde phase dite phase musculaire ne devient opérante qu'une fois la première phase vasculaire obtenue. La contraction des muscles ischio-caverneux (IC) entourant le corps caverneux (CC) fait monter la pression intra caverneuse (PIC) de 100m/hg à 400, ou 600mm/hg voir plus, ce qui constitue la rigidité pénienne. Cette élévation importante de la pression intra caverneuse (PIC) n'est possible que grâce à l'arrêt de la dilatation du corps caverneux (CC) par l'albuginée.

Pour obtenir une érection rigide, ayant donc une pression intra caverneuse (PIC) élevée due à la contraction des muscles ischio-caverneux (IC), la phase vasculaire doit précéder la phase musculaire.

En effet, si la phase vasculaire n'est pas achevée la tension s'exerçant sur l'albuginée est faible et en conséquence son élasticité importante. La contraction musculaire est alors peu ou pas efficace.

Ceci s'explique et se mesure par le module de Young du corps caverneux (CC) et de l'albuginée.

Le module de Young nous indique que lorsqu'une faible tension est exercée sur l'albuginée son élasticité est importante, alors que pour une tension importante l'élasticité diminue.

Si la pression intra caverneuse (PIC) est faible, le module de Young de l'albuginée est faible, entraînant une faible variation de la pression intra caverneuse (PIC), (delta P-IC), provoquée par la contraction des muscles ischio-caverneux (IC), car la variation de pression (delta P-IC) est absorbée par l'élasticité des tissus.

Par contre, à la fin de la phase vasculaire, si la tumescence est complète, la tension s'exerçant sur l'albuginée est forte, son élasticité faible, le module de Young est élevé, provoquant une variation de pression (delta P-IC) qui est faiblement absorbé par l'élasticité des tissus et donc des variations de la pression intra caverneuse (PIC) importantes.

Les trois causes les plus fréquentes d'impuissance sont :
- Les causes artérielles dues à la diminution du débit d'entrée,
- Les fuites dues à l'absence de diminution du débit de sortie, ces deux causes étant des perturbations des débits artério-veineux (DAV),
- Les causes musculaires dues à une diminution de la force musculaire des muscles ischio-caverneux (IC).

Le dispositif de mesure 1, comprenant soit un capteur 2 constitué d'un brassard 12 soit un capteur formé d'un anneau 50, permet de répondre aux besoins énoncés ci-dessus.

### Les causes artérielles ou mesure du débit d'entrée

La mesure du débit d'entrée se fait classiquement à partir de l'effet Doppler. L'effet Doppler mesure la vitesse du sang mais ne mesure pas le débit, à moins de connaître le diamètre des vaisseaux, ce qui est possible mais peu précis. Il est possible de mesurer des variations de débit en mesurant la surface sous la courbe du signal Doppler, mais ce ne sont que des variations.

De plus, les mesures par effet Doppler ne sont pas adaptées aux mesures en continu car la présence de l'investigateur s'impose en permanence. Ceci est dû au fait que la position de la sonde Doppler doit être maintenue avec un angle précis par rapport à l'artère, le moindre mouvement faisant « perdre » le signal.

Le Doppler est donc mal adapté aux mesures en continu surtout au cours du sommeil.

C'est la raison pour laquelle le dispositif de mesure 1 suivant la présente invention permet d'assurer des mesures en continu notamment au cours du sommeil du débit d'entrée par la mesure de la pression du pouls (PP) et du volume du pouls (VP).

Le dispositif de mesure 1 permet de mesurer les débits artério-veineux (DAV), à savoir le débit des artères caverneuses ou débit d'entrée. Cette méthode est basée sur la mesure de la pression du pouls (PP). En effet, nous observons au cours des enregistrements nocturnes ou diurnes, après injection d'un vasodilatateur, que les variations de pression engendrées par le pouls sont visibles. Ces variations de pression sont corrélées avec le débit sanguin dans certaines conditions.

Le dispositif de mesure 1 permet de mesurer les débits artério-veineux (DAV) à savoir le débit d'entrée en continu selon deux méthodes différentes :
- Soit par volume du pouls (VP) ce qui demande deux capteurs différents, l'un de pression pour mesurer la pression intra caverneuse (PIC) et l'autre de photopléthysmographie (PPL) pour mesurer le débit.
- Soit par pression du pouls (PP) avec un seul capteur qui mesure simultanément la pression intra caverneuse (PIC) et les variations de pression de la pression du pouls (PP).

### Les fuites dues à l'absence de diminution du débit de sortie

Les fuites veineuses ou fuites caverneuses ou fuites cavemo-veineuses sont les principales causes de la diminution du débit de sortie.

Ceci est dû à une diminution de l'élasticité ou compliance du corps caverneux (CC) qui gène sa dilatation (donc l'érection), mais surtout entrave la diminution des débits artério-veineux (DAV) et plus particulièrement du débit de sortie.

En effet, le réseau veineux du corps caverneux (CC), qui permet le débit de sortie, est à la périphérie du corps caverneux (CC) sous l'albuginée, il est donc situé entre le corps caverneux (CC) et l'albuginée.

Lors de la dilatation du corps caverneux (CC) dont la compliance est forte, le réseau veineux se trouve comprimé entre le corps caverneux (CC) et l'albuginée dont la compliance est faible.

Si pour des raisons pathologiques, la compliance du corps caverneux (CC) diminue, la compression veineuse ne peut se faire. Les débits artério-veineux (DAV), et plus particulièrement le débit de sortie, ne sont pas réduits, on parle alors de fuite veineuse qui est en fait une fuite caverno-veineuse non pas liée à une pathologie veineuse mais bien à une pathologie caverneuse.

Dans ce cas, même si le débit d'entrée est suffisant, la pression intra caverneuse (PIC) ne peut monter si la fuite est importante. En cas de fuite faible, le débit d'entrée peut compenser cette perte par une augmentation de débit.

Le dispositif de mesure 1 permet de mesurer la fuite à partir de la courbe de la pression intra caverneuse (PIC).

La courbe illustrée en figure 7 représente la pression intra caverneuse (PIC), dont la ligne de base indique le niveau de la pression intra caverneuse (PIC) en dehors d'une contraction des muscles ischio-caverneux (IC). L'élévation brutale de la pression correspond à une contraction des muscles ischio-caverneux (IC) qui est parfaitement corrélée avec l'activité électromyographique des muscles ischio-caverneux (IC).

Au cours du sommeil ces contractions sont involontaires, au cours d'une séance de rééducation elles sont volontaires, mais l'allure de la courbe est identique.

Le dispositif de mesure 1 permet de mettre en évidence, une chute de la pression intra caverneuse (PIC) en dessous de la ligne de base juste après une contraction volontaire (contraction musculaire), puis une remontée en quelques secondes pour retrouver le niveau de pression obtenu juste avant la contraction.

En effet, lorsque la pression intra caverneuse (PIC) est stable la valeur de la pression intra caverneuse (PIC) n'est autre que la pression systolique moyenne, les débits artério-veineux (DAV), et plus particulièrement le débit d'entrée, sont alors égaux aux débits de sortie puisque la pression intra caverneuse (PIC) est stable permettant de mesurer le débit de sortie.

La chute brutale de la pression intra caverneuse (PIC) est provoquée par la contraction musculaire. En effet, en dehors de ces contractions, on n'observe jamais de chute brutale de la pression intra caverneuse (PIC). Cette chute de pression est due à une légère diminution du volume de sang contenu dans le corps caverneux (CC).

Il s'agit donc d'une fuite provoquée par la contraction musculaire qui chasse un volume de sang quantifiable. En effet, nous pouvons compter le nombre de battements cardiaques nécessaires à son comblement, connaissant le volume du pouls nous pouvons en déduire le volume de la fuite lors d'une contraction.

Pour un patient normal, la compliance du corps caverneux (CC) est élevée et la pression intra caverneuse (PIC) comprime le réseau veineux, suffisamment pour permettre aux débits artério-veineux (DAV), et plus particulièrement aux débits de sortie, de rester égaux aux débits d'entrée, c'est la fuite physiologique.

Au cours d'une contraction des muscles ischio-caverneux (IC), la brusque élévation de la pression intra caverneuse (PIC) est due à une compression extrinsèque de l'albuginée par les muscles ischio-caverneux IC. Cette pression se transmet bien sur la face interne de l'albuginée puis aux corps caverneux (CC), ce qui provoque une élévation brutale de la pression intra caverneuse (PIC) et une forte compression du réseau veineux.

Par contre, si la compliance est faible, la transmission des pressions n'est pas suffisante pour obtenir une compression complète des veines, ce qui chasse un volume de sang en dehors du corps caverneux (CC).

La mesure de la fuite se fait à partir de la surface du triangle observée juste après une contraction musculaire volontaire.

Pour un patient pathologique, surtout lorsqu'on suspecte une fuite, la mesure du module de Young du corps caverneux (CC) devient fondamentale car elle permet d'évaluer la compliance du corps caverneux (CC) et donc de confirmer la cause de la fuite.

Il existe plusieurs techniques pour effectuer la mesure du module de Young du corps caverneux (CC). Bien évidemment, cette mesure, ainsi que l'évaluation de la compliance du corps caverneux (CC) peuvent être réalisées avec le dispositif de mesure et de contrôle 1 suivant la présente invention.

### Les causes musculaires dues à une diminution de la force musculaire des muscles ischio-caverneux (IC)

La contraction musculaire est bien visible sur la courbe illustrée en figure 7, il s'agit bien d'une contraction musculaire parfaitement corrélée avec l'activité électro-miographique EMG des muscles ischio-caverneux (IC).

Il existe même un lien de causalité entre les pics enregistrés et la contraction des muscles ischio-caverneux (IC). La contraction des muscles ischio-caverneux (IC) constitue la rigidité pénienne.

Le dispositif de mesure et de contrôle 1 suivant la présente invention permet, soit la nuit soit au cours d'une érection artificielle, de réaliser les mesures suivantes :
- **Le Pmax** : contraction maximum,
- **Le delta P** : le gradient de pression enregistré au-dessus de la ligne de base,
- **Le delta Moyen au** cours d'une séance ou d'une nuit,
- **La surface** sous la courbe de chaque contraction objectivant le travail fourni par le muscle et la moyenne de ses surfaces,
- **Le nombre de pics**,
- **La largeur de chaque pic** mesurée à sa base et leur moyenne,
- **La mesure de la fuite**,
- **Les débits d'entrée et de sortie**,
- **Les débits artério-veineux (DAV)**.

### PROCÉDURE DE L'ENREGISTREMENT NOCTURNE

Le dispositif de mesure 1 constitué du capteur 2, 50 suivant la présente invention permet la surveillance des patients comportant des dysfonctions érectiles, ces patients ayant été diagnostiqués grâce à la mesure de leurs érections nocturnes.

La mesure des érections nocturnes est essentielle pour déterminer le type d'impuissance, à savoir les impuissances psychogènes versus les impuissances organiques.

Le dispositif de mesure 1 utilisé pour les mesures nocturnes est pratiquement le même que celui décrit préalablement, il diffère du fait qu'il n'est pas relié à un écran d'ordinateur 18, que les données sont stockées sur une mémoire amovible, et qu'il n'y a pas de système d'électro-stimulation.

Le logiciel d'analyse est aussi un peu différent, car il calcule notamment la durée des érections, leur nombre et établit des zooms sur chaque érection.

L'enregistrement se fait à domicile sans enregistrement de sommeil mais peut aussi avoir lieu en laboratoire de sommeil.

Si l'enregistrement se fait à domicile, le boîtier 35 est confié au patient qui vient le chercher chez le médecin.

Au préalable, le médecin aura programmé le boîtier 35 afin d'enregistrer les caractéristiques du patient (date, nom, prénom, age, tabac, pathologies à détailler, glycémie, cholestérol, médications, et toutes autres informations susceptibles d'intervenir sur sa fonction érectile).

Le médecin, après avoir enregistré les données du patient vérifie la charge des piles, le calibrage, et le bon fonctionnement de l'enregistrement. Il déconnecte le boîtier 35 de son ordinateur et le confie au patient accompagnés d'un mémo explicatif du branchement.

Au coucher, le patient place le brassard 12 solidaire du réservoir 3 sur son pénis P. Le réservoir 3 est relié par les tubulures 10, 11 et 20 et les vannes trois voies 9, 13 et 17 au capteur de pression 21 qui est connecté au boîtier 35. Les tubulures 10, 11 et 20 sont prévues d'une longueur d'au moins un mètre permettant au patient de placer l'ensemble à proximité sur sa table de nuit ou au niveau du sol.

Une fois couché, le patient met en route le dispositif de mesure 1 à l'aide du bouton marche arrêt prévu sur le boîtier 35, une LED rouge clignote indiquant que l'enregistrement a commencé. Un zéro automatique du capteur de pression 21 est réalisé par le logiciel après quelques minutes d'enregistrement.

Au réveil, le patient vérifie que la LED est toujours allumée attestant du fait que l'enregistrement n'ait pas été interrompu au cours de la nuit. Il met le boîtier 35 sur arrêt.

Le patient recommence les mêmes opérations décrites ci-dessus les nuits suivantes.

Après trois nuits consécutives, le patient ramène le dispositif de mesure 1 chez le médecin qui bascule les données contenues sur une mémoire amovible disposée dans le boîtier 35 sur son ordinateur. Les données sont analysées partiellement par le logiciel puis envoyées via Internet dans la base de données du centre d'analyse et de télé contrôle (CAT). Une sortie papier des courbes de chaque nuit, des zooms et des calculs sera imprimées puis l'analyse complète sera envoyée par le télé contrôle (CAT).

Cette procédure d'enregistrement nocturne peut être réalisé avec le dispositif de mesure 1 constitué de l'anneau ouvert ou semi ouvert 50.

### PROCÉDURE POUR LA MESURE ET/OU LA SURVEILLANCE DE LA REEDUCATION DES DYSFONCTIONS ERECTILES

Le dispositif de mesure 1 constitué du capteur 2, 50 suivant la présente invention permet une mesure de l'évolution et/ou une surveillance de la rééducation des patients comportant des dysfonctions érectiles. Cette mesure de l'évolution et/ou la surveillance de la rééducation peut être réalisée soit au cabinet du médecin traitant soit au domicile du patient.

Lorsque le diagnostic d'impuissance organique est établi la nuit par le dispositif de mesure 1, ce dernier permet aussi la mesure de l'évolution et/ou la surveillance de la rééducation grâce à quelques modifications du logiciel.

La mesure de l'évolution et/ou la surveillance de la rééducation est indiquée spécialement pour les patients présentant une pathologie musculaire ayant une phase vasculaire normale, mais aussi pour ceux ayant une pathologie mixte vasculaire d'origine artérielle ou veineuse et musculaire.

Cette mesure de l'évolution et/ou la surveillance de la rééducation peut être réalisée soit au moyen du brassard 12, soit au moyen de l'anneau ouvert ou semi ouvert 50 muni des électrodes 56 décrit précédemment permettant d'assurer une meilleure mesure. A titre d'exemple non limitatif seule la procédure avec le brassard 12 a été décrite sachant qu'elle sera sensiblement identique à celle de l'anneau 50.

Le patient est placé sur un lit d'examen ou sur son lit, les électrodes 22 raccordées au boîtier 35 sont placées sur la partie moyenne du pénis P, l'une à droite et l'autre à gauche, elles peuvent aussi être placées sur diverses régions du pénis en particulier le long du nerf dorsal en dehors du brassard 12.

Le réservoir 3 du brassard 12 a été rempli au préalable par le médecin de 2CC d'eau, par l'intermédiaire de la seringue, tandis que le capteur de pression 21 est raccordé au boîtier 35. Le patient n'ayant pas à manipuler la seringue, cette dernière restera chez le praticien.

Le boîtier 35 contient un amplificateur, un convertisseur A/D, un microprocesseur, et un système d'électrostimulation.

Le boîtier 35 est relié par un câble USB ou par un système bluetooth ou par un autre système à l'ordinateur 18. Le dispositif est allumé, et le médecin procède à la mise à zéro du capteur de pression 21, soit automatiquement après une minute de fonctionnement, soit à l'aide d'un bouton virtuel situé sur l'écran soit de l'ordinateur 18, soit d'un PDA, soit d'un téléphone portable permettant de piloter le boîtier 35 (arrêt, marche, zéro, électro-stimulation).

Le système d'électro-stimulation du boîtier 35 est mis en route et le patient règle l'intensité de la stimulation, soit à l'aide d'un potentiomètre placé sur le boîtier 35 soit à l'aide d'un bouton virtuel situé sur l'écran, soit de l'ordinateur 18, soit d'un PDA, soit d'un téléphone portable.

Une injection intra caverneuse d'une vasodilatation est alors pratiquée chez tous les patients dont l'érection n'est pas suffisante suite à une stimulation du réflexe vasculaire pour provoquer une érection. Cette injection participe aussi à l'amélioration de la composante artérielle.

L'électro-stimulation se poursuit jusqu'à la fin de la séance, comme dans toute rééducation musculaire car elle améliore les performances de la rééducation des muscles ischio-caverneux (IC), ainsi que la vasodilatation des artères caverneuses.

Le patient doit alors vérifier que ses contractions volontaires provoquent bien une élévation de la pression intra caverneuse (PIC), ce qui prouve que les bons muscles sont contractés car seuls les ischio-caverneux (IC) élèvent la pression intra caverneuse PIC.

Le patient voit alors apparaître à l'écran un masque de contractions musculaires qu'il est invité à suivre, il lui indique la durée et la hauteur optimale de la contraction qui doit être faite ainsi que la durée de la phase de repos.

Ce masque modulable est établi à partir de la courbe de fatigue du muscle ischio-caverneux (IC). Il permet d'optimiser la rééducation. En effet, un travail insuffisant sera synonyme d'absence d'amélioration mais à l'inverse une rééducation trop intense va épuiser les muscles ischio-caverneux (IC). Il existe donc une intensité optimale calculée en fonction de la courbe de fatigue.

La fatigue du muscle ischio-caverneux (IC) se calcule en mesurant l'angle α matérialisé sur la courbe. Pour une largeur de pic donnée, plus l'angle est petit plus la fatigue est importante (fig.7).

Les modifications du gabarit indiquant les modalités de la contraction à effectuer seront calculées soit en temps réel au cours de la séance soit entre deux séances lorsque cette analyse sera faite par le centre d'analyse et de télé contrôle (CAT).

### PROCÉDURE POUR LA MESURE ET/OU LA SURVEILLANCE DE LA REEDUCATION DES PROBLEMES D'EJACULATION PRECOCE

Le dispositif de mesure 1 constitué du capteur 2, 50 suivant la présente invention permet la mesure de l'évolution et/ou la surveillance de la rééducation des patients comportant des problèmes d'éjaculation précoce.

L'éjaculation précoce (EP) touche 30% de la population masculine. Actuellement, le traitement le plus efficace consiste à utiliser certains antidépresseurs qui ont l'avantage d'être efficaces dès le début du traitement mais l'inconvénient de ne pas traiter l'éjaculation précoce (EP).

En effet, à l'arrêt du traitement médicamenteux et dans la grande majorité des cas, le symptôme réapparaît.

Le deuxième traitement efficace utilise un anesthésique local diminuant la sensibilité du gland, son efficacité est réelle mais diminue parfois le plaisir des deux partenaires. Comme pour le traitement chimique, le symptôme réapparaît dès l'arrêt du traitement.

La mesure de l'évolution ou la surveillance de la rééducation péniréale est obtenue au moyen du dispositif de mesure 1 avec électro-stimulation du pénis P par l'intermédiaire des électrodes 22 positionnées sous le réservoir 3 solidaire du brassard 12 ou des électrodes 56 positionnées à l'intérieur de l'anneau 50.

Cette électro-stimulation du pénis P permet une meilleur mesure et de renforcer les muscles ischio-caverneux (IC) entourant le corps caverneux (CC) et de favoriser l'augmentation de la circulation artérielle donc de l'érection nécessaire à la rééducation.

La mesure de l'évolution ou la surveillance de la rééducation peut par exemple être réalisée au domicile du patient qui possède un boîtier 35 qu'il peut connecter via bluetooth à son ordinateur ou à son PDA ou à son téléphone portable, comme pour l'évaluation des dysérections, ou par l'intermédiaire d'un écran solidaire du boîtier 35.

La séance étant conçue de la même manière, la seule chose qui diffère est la façon dont est produite l'érection nécessaire au renforcement musculaire des ischio-caverneux (IC).

Le patient est invité à placer le brassard 12 muni des électrodes 22 sur son pénis P de manière que le réservoir 3 soit en contact avec ce dernier ou l'anneau ouvert 50 comportant ses électrodes 56. Le patient met le boîtier 35 en marche, s'assure que la connexion fonctionne en vérifiant qu'une variation de pression se traduit bien (en appuyant sur le brassard) par une élévation de pression sur l'écran de l'ordinateur.

La stimulation du réflexe vasculaire est obtenue par une variation de la pression sur le gland par des petites compressions entre le pouce et l'index afin de produire une augmentation du débit artériel des artères caverneuses. Cette stimulation du réflexe vasculaire combinée à l'électro-stimulation des muscles ischio-caverneux (IC) produit le plus souvent une érection rigide du pénis P.

Cette stimulation provoque le plus souvent une érection chez les patients atteints d'éjaculation précoce et ceci sans aucune stimulation du réflexe éjaculatoire.

Lorsque la stimulation du réflexe vasculaire ne sera pas suffisante pour déclencher l'érection, l'utilisation de produit vasodilatateur par voie orale ou en injectable, sera instituée.

Il doit d'ailleurs être entendu que la description qui précède n'a été donnée qu'a titre d'exemple et qu'elle ne limite nullement le domaine de l'invention dont on ne sortirait pas en remplaçant les détails d'exécution décrits par tous autres équivalents.

## Revendications

1. Procédé de mesure de la force des muscles ischio-caverneux (IC) provoquant la rigidité du pénis (P) d'un patient et de la variation du débit artériel et veineux assurant l'érection du pénis (P) du patient au moyen d'un dispositif de mesure suivant l'une quelconque des revendications 11 à 25, **caractérisé en ce qu'**il consiste à :
• Placer un capteur (2, 50) autour du pénis (P) comportant des moyens pour capter la pression et les variations de la pression intra caverneuse (PIC) du pénis (P), et au moins un transducteur (21, 55) intégré ou déporté du capteur (2, 50) permettant de transformer le signal de pression issu des moyens pour capter la pression en un signal électrique,
• Et à connecter le capteur (2, 50) à un boîtier (35) comportant des moyens d'amplification et d'enregistrement du signal provenant du transducteur (21, 55), des moyens d'analyse du signal par un microcontrôleur permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

2. Procédé de mesure suivant la revendication 1, **caractérisé en ce qu'**il consiste à mesurer les variations de la pression intra caverneuse (PIC) du pénis (P) afin de quantifier la détumescence, la tumescence et la rigidité de jour comme de nuit du pénis (P).

3. Procédé de mesure suivant la revendication 2, **caractérisé en ce qu'**il consiste à mesurer la pression intra caverneuse (PIC) qui atteint progressivement la pression systolique moyenne du pénis (P).

4. Procédé de mesure suivant la revendication 1, **caractérisé en ce qu'**il consiste à mesurer, pendant la phase vasculaire qui est la première phase de l'érection du pénis (P), l'augmentation du débit artériel d'entrée dans le corps caverneux (CC) et la diminution du débit veineux de sortie.

5. Procédé de mesure suivant la revendication 1, **caractérisé en ce qu'**il consiste à mesurer, pendant la phase musculaire qui est la seconde phase de l'érection du pénis (P), les variations de la contraction et de la rigidité des muscles ischio-caverneux (IC) entourant le corps caverneux (CC).

6. Procédé de mesure suivant la revendication 4, **caractérisé en ce qu'**il consiste à mesurer, en continu, notamment au cours du sommeil du patient, le débit artériel d'entrée et le débit veineux de sortie par la mesure de la pression du pouls (PP) et du volume du pouls (VP).

7. Procédé de mesure suivant la revendication 2, **caractérisé en ce qu'**il consiste à mesurer la fuite cavemo veineuse à partir de la courbe de la pression intra caverneuse (PIC).

8. Procédé de mesure suivant la revendication 1, **caractérisé en ce qu'**il consiste à mesurer la contraction maximum (Pmax), le gradient de pression (Delta P et delta Moyen), la surface sous la courbe de chaque contraction correspondant au travail musculaire, le nombre de pics et la largeur de chaque pic.

9. Procédé de mesure suivant la revendication 1, **caractérisé en ce qu'**il consiste à mesurer la fatigue des muscles ischio-caverneux (IC) en mesurant l'angle (α).

10. Procédé de mesure suivant la revendication 1, **caractérisé en ce qu'**il consiste à mesurer les pics des contractions des muscles ischio-caverneux (IC) et les variations de pression du pouls tout en conservant une pression constante dans le capteur (2, 50) quelle que soit la variation du volume du pénis.

11. Dispositif, de mesure de la force des muscles ischio-caverneux (IC) provoquant la rigidité du pénis (P) d'un patient et de la variation du débit artériel et veineux assurant l'érection du pénis du patient, le dispositif comprenant un capteur (2) constitué :
• D'un brassard (12) solidaire sur sa face interne d'un réservoir (3) rempli d'un liquide permettant de capter la pression et les variations de pression intra caverneuse (PIC),
• D'électrodes de stimulations (22) positionnées entre le réservoir (3) et la peau du pénis (P) du patient,
• Et des tubulures (10, 11, 14, 20) de connexion raccordant le réservoir (3) à un photopléthysmographe (16) et à un transducteur de pression (21) qui sont connectés au boîtier (35) permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

12. Dispositif de mesure suivant la revendication 11, **caractérisé en ce que** le brassard (12) est connecté par l'intermédiaire d'une première vanne trois voies (9) au photopléthysmographe (16) mesurant les déplacements d'une membrane souple (15) solidaire de la troisième tubulure de connexion (14) afin de mesurer le volume du pouls pénien provenant des artères caverneuses.

13. Dispositif de mesure suivant la revendication 11, **caractérisé en ce que** le brassard (12) est connecté par l'intermédiaire de la seconde tubulure de connexion (11) et d'une seconde vanne trois voies (13) à un régulateur de pression (23) pour assurer une pression constante à l'intérieur du réservoir (3) du brassard (12).

14. Dispositif de mesure suivant la revendication 13, **caractérisé en ce que** le régulateur de pression (23) est constitué d'un cylindre (24) comportant deux chambres (25, 26) remplies d'un fluide, séparées par une paroi (27) percée d'un orifice calibré (28) reliant lesdites chambres entre elles, ladite première chambre (25) étant reliée à la vanne trois voies (13), tandis que ladite seconde chambre (26) comporte un piston (29) permettant de faire varier la pression du fluide au moyen d'un dispositif de vis micrométrique (30).

15. Dispositif de mesure suivant la revendication 14, **caractérisé en ce que** la vis micrométrique (30) comporte un ressort (31) disposé autour de la tige de commande du piston (29) afin de régler la tension dudit ressort et maintenir une pression stable dans le brassard (12) tout en permettant des variations de pression élevées et de courte durée.

16. Dispositif de mesure suivant la revendication 11, **caractérisé en ce que** la retenue et la fermeture du brassard (12) autour du pénis du patient sont sécurisées par un lien non élastique (6).

17. Dispositif de mesure suivant la revendication 11, **caractérisé en ce que** le boîtier (35) contient un amplificateur, un convertisseur A/D, un microprocesseur, et une carte mémoire amovible.

18. Dispositif de mesure suivant la revendication 11, **caractérisé en ce que** le photopléthysmographe (16) étant sensible à la pression ce dernier est positionné à une certaine distance du réservoir (3), pour éviter que les variations de pression dudit réservoir viennent perturber ledit photopléthysmographe (16).

19. Dispositif de mesure suivant la revendication 11, **caractérisé en ce que** le photopléthysmographe (16) comporte une diode photoémettrice et une cellule photoélectrique permettant d'envoyer un rayon lumineux en direction de la membrane souple (15) et de recevoir le rayon réfléchi capté par la cellule, tandis que ledit signal reçu par le photopléthysmographie (16) est transmis au boîtier (35) qui permet l'amplification et l'enregistrement dudit signal.

20. Dispositif de mesure de la force des muscles ischio-caverneux (IC) provoquant la rigidité du pénis (P) d'un patient et de la variation du débit artériel et veineux assurant l'érection du pénis du patient, le dispositif comprenant un capteur (2) constitué :
• D'un anneau ouvert ou semi ouvert (50) comportant deux branches (51, 52) en arc de cercle reliées entre elles par un élément souple (53), lesdites branches (51, 52) comportant sur leur face interne des zones de contacts constituées par des pastilles en caoutchouc (54) présentant une faible déformation élastique et venant prendre appui contre le pénis (P) du patient de manière à décaler légèrement la face interne desdites branches de ce dernier.
• D'une jauge de contrainte (55) solidaire de l'élément souple (53) et connectée au boîtier (35) permettant de mesurer l'état de fatigue des muscles ischio-caverneux (IC), de mesurer les variations de la force musculaire des muscles ischio-caverneux (IC) et de mesurer le module de Young du corps caverneux (CC) permettant d'évaluer la compliance dudit corps caverneux.

21. Dispositif de mesure suivant la revendication 20, **caractérisé en ce que** les branches (51, 52) sont plates en forme de lamelle et réalisées dans un matériau ayant une grande résistance à la déformation élastique ou dit peu déformable.

22. Dispositif de mesure suivant la revendication 20, **caractérisé en ce que** l'élément souple (53), reliant les branches (51, 52) est constitué d'une fine platine de métal présentant des caractéristiques de déformation élastique importante.

23. Dispositif de mesure et/ou de rééducation suivant la revendication 20, **caractérisé en ce que** l'anneau ouvert (50) comporte des moyens d'électro stimulation qui sont constitués d'électrodes (56) disposées sur les pastilles en caoutchouc (54).

## Claims

1. A method for measuring the strength of the ischiocavernous muscles (IC) causing the stiffness of the penis (P) of a patient and the variation of the arterial and venous circulation ensuring the erection of the penis (P) of the patient using a measuring device according to any one of claims 11 to 25, **characterized in that** it consists of:
• Placing a sensor (2, 50) around the penis (P) comprising means for sensing the pressure and the variations of the intracavernous pressure (PIC) of the penis (P), and at least one transducer (21, 55) integrated into or offset from the sensor (2, 50) making it possible to convert the pressure signal from the pressure sensing means into an electric signal,
• And connecting the sensor (2, 50) to a casing (35) that comprises means for amplifying and recording the signal coming from the transducer (21, 55), means for analyzing the signal using a microcontroller making it possible to measure the state of fatigue of the ischiocavernous muscles (IC), to measure the variations of the muscle strength of the ischiocavernous muscles (IC), and to measure the Young's modulus of the cavernous body (CC) making it possible to evaluate the compliance of said cavernous body.

2. The measuring method according to claim 1, **characterized in that** it consists of measuring the variations of the intracavernous pressure (PIC) of the penis (P) so as to quantify the detumescence, tumescence and stiffness of the penis (P) both day and night.

3. The measuring method according to claim 2, **characterized in that** it consists of measuring the intracavernous pressure (PIC) that gradually reaches the average systolic pressure of the penis (P).

4. The measuring method according to claim 1, **characterized in that** it consists of measuring, during the vascular phase, which is the first phase of the erection of the penis (P), the increase in the arterial input circulation in the cavernous body (CC) and the decrease of the venous output circulation.

5. The measuring method according to claim 1, **characterized in that** it consists of measuring, during the muscular phase, which is the second phase of the erection of the penis (P), the variations of the contraction and the stiffness of the ischiocavernous muscles (IC) surrounding the cavernous body (CC).

6. The measuring method according to claim 4, **characterized in that** it consists of continuously measuring, in particular while the patient is sleeping, the arterial input circulation and the venous output circulation by measuring the pulse pressure (PP) and the pulse volume (VP).

7. The measuring method according to claim 2, **characterized in that** it consists of measuring the cavernous venous leak from the intracavernous pressure (PIC) curve.

8. The measuring method according to claim 1, **characterized in that** it consists of measuring the maximum contraction (Pmax), the pressure gradient (Delta P and Average delta), the surface under the curve of each contraction corresponding to the muscular work, the number of peaks, and the width of each peak.

9. The measuring method according to claim 1, **characterized in that** it consists of measuring the fatigue of the ischiocavernous muscles (IC) by measuring the angle (α).

10. The measuring method according to claim 1, **characterized in that** it consists of measuring the peaks of the contractions of the ischiocavernous muscles (IC) and the pressure variations of the pulse while keeping a constant pressure in the sensor (2, 50) irrespective of the variation of the volume of the penis.

11. A device for measuring the strength of the ischiocavernous muscles (IC) causing the stiffness of the penis (P) of a patient and the variation of the arterial and venous circulation ensuring the erection of the penis of the patient, the device including a sensor (2) made up of:
• An armband (12), the inner surface of which is secured to a reservoir (3) filled with a liquid making it possible to sense the pressure and the intracavernous pressure (PIC) variations,
• Stimulating electrodes (22) positioned between the reservoir (3) and the skin of the penis (P) of the patient,
• And connecting tubes (10, 11, 14, 20) connecting the reservoir (3) to a photoplethysmograph (16) and a pressure transducer (21) that are connected to the casing (35), making it possible to measure the state of fatigue of the ischiocavernous muscles (IC), to measure the variations of the muscle strength of the ischiocavernous muscles (IC), and to measure the Young's modulus of the cavernous body (CC), making it possible to evaluate the compliance of said cavernous body.

12. The measuring device according to claim 11, **characterized in that** the armband (12) is connected by means of a first three-way valve (9) to the photoplethysmograph (16) measuring the displacements of a flexible membrane (15) secured to the third connecting tube (14) so as to measure the volume of the penile pulse coming from the cavernous arteries.

13. The measuring device according to claim 11, **characterized in that** the armband (12) is connected by means of the second connecting tube (11) and a second three-way valve (13) to a pressure regulator (23) to provide constant pressure inside the reservoir (3) of the armband (12).

14. The measuring device according to claim 13, **characterized in that** the pressure regulator (23) is made up of a cylinder (24) comprising two chambers (25, 26) filled with a fluid, separated by a wall (27) pierced with a calibrated opening (28) connecting said chambers to one another, said first chamber (25) being connected to the three-way valve (13), while said second chamber (26) comprises a piston (29) making it possible to vary the pressure of the fluid using a micrometric screw device (30).

15. The measuring device according to claim 14, **characterized in that** the micrometric screw (30) comprises a spring (31) arranged around the control rod of the piston (29) so as to adjust the tension of said spring and maintain a stable pressure in the armband (12) while allowing high and short-lasting pressure variations.

16. The measuring device according to claim 11, **characterized in that** the retention and closing of the armband (12) around the penis of the patient are secured by a non-elastic link (6).

17. The measuring device according to claim 11, **characterized in that** the casing (35) contains an amplifier, an A/D converter, a microprocessor, and a removable memory card.

18. The measuring device according to claim 11, **characterized in that**, the photoplethysmograph (16) being pressure-sensitive, the latter is positioned at a certain distance from the reservoir (3), to prevent the pressure variations of said reservoir from disrupting said photoplethysmograph (16).

19. The measuring device according to claim 11, **characterized in that** the photoplethysmograph (16) comprises a light-emitting diode and a photoelectric cell making it possible to send a light ray toward the flexible membrane (15) and to receive the reflected ray captured by the cell, while said signal received by photoplethysmography (16) is transmitted to the casing (35), which makes it possible to amplify and record said signal.

20. A device for measuring the strength of the ischiocavernous muscles (IC) causing the stiffness of the penis (P) of a patient and the variation of the arterial and venous circulation ensuring the erection of the penis of the patient, the device including:
• An open or semi-open ring (50) comprising two branches (51, 52) in an arc of circle connected to one another by a flexible element (53), said branches (51, 52) comprising, on the inner surface thereof, contact zones made up of rubber pellets (54) with a small elastic deformation and bearing against the penis (P) of the patient so as to slightly offset the inner surface of said branches therefrom.
• A strain gauge (55) secured to the flexible element (53) and connected to the casing (35), making it possible to measure the state of fatigue of the ischiocavernous muscles (IC), measure the variations of the muscle strength of the ischiocavernous muscles (IC), and measure the Young's modulus of the cavernous body (CC), making it possible to evaluate the compliance of said cavernous body.

21. The measuring device according to claim 20, **characterized in that** the branches (51, 52) are flat and strip-shaped and are made from a material having a high resistance to elastic deformation or said to be only slightly deformable.

22. The measuring device according to claim 20, **characterized in that** the flexible element (53), connecting the branches (51, 52), is made up of a thin metal platen having significant elastic deformation characteristics.

23. The measuring and/or rehabilitation device according to claim 20, **characterized in that** the open ring (50) comprises electrostimulation means made up of electrodes (56) positioned on the rubber pellets (54).

## Patentansprüche

1. Messverfahren der Kraft der ischiokavernösen Muskeln (IC), die die Steifheit des Penis (P) eines Patienten hervorrufen, und der Schwankung des arteriellen und venösen Durchflusses, der die Erektion des Penis (P) des Patienten mit einer Messvorrichtung nach einem der Ansprüche 11 bis 25 sichert, **dadurch gekennzeichnet, dass** es darin besteht:
• einen Sensor (2, 50) um den Penis (P) zu platzieren, der Mittel aufweist, um den Druck und die Schwankungen des intrakavernösen Drucks (PIC) des Penis (P) zu erfassen, und mindestens einen im Sensor (2, 50) integrierten oder von diesem versetzten Umformer (21, 55), der es erlaubt, das von den Mitteln zur Druckerfassung ausgegebene Drucksignal in ein elektrisches Signal zu verwandeln,
• und den Sensor (2, 50) mit einem Gehäuse (35) zu verbinden, das Mittel zur Verstärkung und Aufzeichnung des vom Umformer (21, 55) kommenden Signals, Mittel zur Analyse des Signals durch einen Mikrocontroller umfasst, wodurch es erlaubt wird, den Ermüdungszustand der ischiokavernösen Muskeln (IC) zu messen, die Schwankungen der Muskelkraft der ischiokavernösen Muskeln (IC) zu messen und das Young-Modul des kavernösen Körpers (CC) zu messen, was ermöglicht, die Compliance des kavernösen Körpers zu beurteilen.

2. Messverfahren nach Anspruch 1, **dadurch gekennzeichnet, dass** es darin besteht, die Schwankungen des intrakavernösen Drucks (PIC) des Penis (P) zu messen, um tags wie nachts die Detumeszenz, die Tumeszenz und die Steifheit des Penis (P) zu quantifizieren.

3. Messverfahren nach Anspruch 2, **dadurch gekennzeichnet, dass** es darin besteht, den intrakavernösen Druck (PIC) zu messen, der schrittweise den durchschnittlichen systolischen Druck des Penis (P) erreicht.

4. Messverfahren nach Anspruch 1, **dadurch gekennzeichnet, dass** es darin besteht, während der vaskulären Phase, die die erste Phase der Erektion des Penis (P) ist, die Erhöhung des arteriellen Durchflusses im kavernösen Körper (CC) und die Verringerung des ausgehenden venösen Durchflusses zu messen.

5. Messverfahren nach Anspruch 1, **dadurch gekennzeichnet, dass** es darin besteht, während der muskulären Phase, die die zweite Phase der Erektion des Penis (P) ist, die Schwankungen der Kontraktion und der Steifheit der ischiokavernösen Muskeln (IC) zu messen, die den kavernösen Körper (CC) umschließen.

6. Messverfahren nach Anspruch 4, **dadurch gekennzeichnet, dass** es darin besteht, vor allem im Schlaf des Patienten den eingehenden arteriellen Durchfluss und den ausgehenden venösen Durchfluss durch Messen des Pulsdrucks (PP) und des Pulsvolumens (VP) kontinuierlich zu messen.

7. Messverfahren nach Anspruch 2, **dadurch gekennzeichnet, dass** es darin besteht, ausgehend von der Kurve des intrakavernösen Drucks (PIC) den kavernovenösen Verlust zu messen.

8. Messverfahren nach Anspruch 1, **dadurch gekennzeichnet, dass** es darin besteht, die maximale Kontraktion (Pmax), den Druckgradienten (Delta P und Delta Durchschnitt), die Fläche unter der Kurve jeder Kontraktion, die der Muskelarbeit entspricht, die Anzahl der Peaks und die Breite jedes Peaks zu messen.

9. Messverfahren nach Anspruch 1, **dadurch gekennzeichnet, dass** es darin besteht, die Ermüdung der ischiokavernösen Muskeln (IC) durch Messen des Winkels (α) zu messen.

10. Messverfahren nach Anspruch 1, **dadurch gekennzeichnet, dass** es darin besteht, unabhängig von der Schwankung des Volumens des Penis die Peaks der Kontraktionen der ischiokavernösen Muskeln (IC) und die Schwankungen des Pulsdrucks bei Beibehaltung eines konstanten Drucks im Sensor (2, 50) zu messen.

11. Messvorrichtung der Kraft der ischiokavernösen Muskeln (IC), die die Steifheit des Penis (P) eines Patienten hervorrufen, und der Schwankung des arteriellen und venösen Durchflusses, der die Erektion des Penis des Patienten sichert, wobei die Vorrichtung einen Sensor (2) umfasst, der besteht:
• aus einer Binde (12), die auf ihrer Innenseite mit einem Reservoir (3) verbunden ist, das mit einer Flüssigkeit gefüllt ist, die es erlaubt, den Druck und die Schwankungen des intrakavernösen Drucks (PIC) zu erfassen,
• aus Stimulationselektroden (22), die zwischen dem Reservoir (3) und der Haut des Penis (P) des Patienten positioniert sind,
• und aus Verbindungsrohren (10, 11, 14, 20), die das Reservoir (3) mit einem Photoplethysmographen (16) und einem Druckumformer (21) verbinden, die mit dem Gehäuse (35) verbunden sind, wodurch es erlaubt wird, den Ermüdungszustand der ischiokavernösen Muskeln (IC) zu messen, die Schwankungen der Muskelkraft der ischiokavernösen Muskeln (IC) zu messen und das Young-Modul des kavernösen Körpers (CC) zu messen, was erlaubt, die Compliance des kavernösen Körpers zu beurteilen.

12. Messvorrichtung nach Anspruch 11, **dadurch gekennzeichnet, dass** die Binde (12) über ein erstes Dreiwegeventil (9) mit dem Photoplethysmographen (16) verbunden ist, der die Verschiebungen einer elastischen Membran (15) misst, die mit dem dritten Verbindungsrohr (14) verbunden ist, um das Volumen des Penispulses von den kavernösen Arterien zu messen.

13. Messvorrichtung nach Anspruch 11, **dadurch gekennzeichnet, dass** die Binde (12) über das zweite Verbindungsrohr (11) und ein zweites Dreiwegeventil (13) mit einem Druckregler (23) verbunden ist, um einen konstanten Druck im Reservoir (3) der Binde (12) zu gewährleisten.

14. Messvorrichtung nach Anspruch 13, **dadurch gekennzeichnet, dass** der Druckregler (23) aus einem Zylinder (24) besteht, der zwei mit einer Flüssigkeit gefüllte Kammern (25, 26) aufweist, die durch eine Wand (27) mit einer kalibrierten Öffnung (28) getrennt sind, die die Kammern miteinander verbindet, wobei die erste Kammer (25) mit dem Dreiwegeventil (13) verbunden ist, wogegen die zweite Kammer (26) einen Kolben (29) aufweist, der es erlaubt, den Druck des Fluids mit Hilfe einer Mikrometerschraubenvorrichtung (30) zu verändern.

15. Messvorrichtung nach Anspruch 14, **dadurch gekennzeichnet, dass** die Mikrometerschraube (30) eine Feder (31) aufweist, die um den Steuerstab des Kolbens (29) angeordnet ist, um den Druck der Feder zu regeln und um einen stabilen Druck in der Binde (12) aufrechtzuerhalten und dabei hohe Druckschwankungen kurzer Dauer zu erlauben.

16. Messvorrichtung nach Anspruch 11, **dadurch gekennzeichnet, dass** der Halt und der Verschluss der Binde (12) um den Penis des Patienten durch eine nicht elastische Verbindung (6) abgesichert ist.

17. Messvorrichtung nach Anspruch 11, **dadurch gekennzeichnet, dass** das Gehäuse (35) einen Verstärker, einen A/D-Umformer, einen Mikroprozessor und eine entfernbare Speicherkarte enthält.

18. Messvorrichtung nach Anspruch 11, **dadurch gekennzeichnet, dass** der Photoplethysmograph (16), der für den Druck dieses Letzten sensibel ist, in einem bestimmten Abstand vom Reservoir (3) angeordnet ist, um zu vermeiden, dass die Druckschwankungen des Reservoirs den Photoplethysmographen (16) beeinflussen.

19. Messvorrichtung nach Anspruch 11, **dadurch gekennzeichnet, dass** der Photoplethysmograph (16) eine fotoemissive Diode und eine fotoelektrische Zelle aufweist, die es erlauben, einen Lichtstrahl in Richtung der elastischen Membran (15) zu senden und den reflektierten, von der Zelle aufgefangenen Strahl zu empfangen, wogegen das durch den Photoplethysmographen (16) empfangene Signal zum Gehäuse (35) übertragen wird, das die Verstärkung und die Aufzeichnung des Signals erlaubt.

20. Messvorrichtung der Kraft der ischiokavernösen Muskeln (IC), die die Steifheit des Penis (P) eines Patienten hervorrufen, und der Schwankung des arteriellen und venösen Durchflusses, der die Erektion des Penis des Patienten sichert, wobei die Vorrichtung einen Sensor (2) umfasst, der besteht aus:
• einem offenen oder halboffenen Ring (50), der zwei kreisförmige Arme (51, 52) aufweist, die miteinander durch ein elastisches Element (53) verbunden sind, wobei die Arme (51, 52) auf ihrer Innenseite Kontaktzonen aufweisen, die von Gummiplättchen (54) gebildet werden, die eine geringe elastische Verformung aufweisen und sich auf dem Penis (P) des Patienten derart abstützen, dass die Innenseite der Arme dieses Letzten leicht verschoben werden,
• einem Dehnungsmesser (55), der mit dem elastischen Element (53) verbunden ist und an das Gehäuse (35) angeschlossen ist, wodurch es erlaubt wird, den Ermüdungszustand der ischiokavernösen Muskeln (IC) zu messen, die Schwankungen der Muskelkraft der ischiokavernösen Muskeln (IC) zu messen und das Young-Modul des kavernösen Körpers (CC) zu messen, was erlaubt, die Compliance des kavernösen Körpers zu beurteilen.

21. Messvorrichtung nach Anspruch 20, **dadurch gekennzeichnet, dass** die Arme (51, 52) lamellenförmig flach und aus einem Material hergestellt sind, das gegenüber elastischer Verformung eine hohe Festigkeit besitzt oder wenig verformbar ist.

22. Messvorrichtung nach Anspruch 20, **dadurch gekennzeichnet, dass** das elastische Element (53), das die Arme (51, 52) verbindet, aus einem dünnen Metallplättchen besteht, das eine hohe elastische Verformbarkeit aufweist.

23. Mess- und/oder Rehabilitationsvorrichtung nach Anspruch 20, **dadurch gekennzeichnet, dass** der offene Ring (50) Elektrostimulationsmittel aufweist, die von Elektroden (56) gebildet werden, die auf den Gummiplättchen (54) angeordnet sind.
